Europäisches Patentamt

European Patent Office

Office européen des brevets

(11) Publication number: **0 596 449 A2**

## EUROPEAN PATENT APPLICATION

(21) Application number: **93117728.1**

(22) Date of filing: **02.11.93**

(51) Int. Cl.5: **A61K 31/00, A61K 31/505, A61K 31/165, A61K 31/48**

(30) Priority: **04.11.92 US 971437**

(43) Date of publication of application:
**11.05.94 Bulletin 94/19**

(84) Designated Contracting States:
**AT BE CH DE DK ES FR GB GR IE IT LI LU MC NL PT SE**

(71) Applicant: **E.R. SOUIBB & SONS, INC.**
**P.O.Box 4000**
**Princeton New Jersey 08543-4000(US)**

(72) Inventor: **Grover, Gary J.**
**101 Bowne Station Road**
**Stockton, NJ(US)**
Inventor: **Antonaccio, Michael J.**
**11 Douglas Drive-R.D. No.4**
**Princeton, NJ(US)**

(74) Representative: **VOSSIUS & PARTNER**
**Siebertstrasse 4**
**D-81675 München (DE)**

(54) Serotonin receptor antagonists for treating myocardial ischemia.

(57) A method for treating myocardial ischemia in a mammalian species by administrating a serotonin receptor antagonist such as cinanserin, ketanserin or LY 53857.

This invention relates to the use of a serotonin receptor antagonist, such as cinanserin, ketanserin and LY 53857 for the preparation of a pharmaceutical composition useful in treating myocardial ischemia in mammalian species.

In accordance with the present invention, the use of a serotonin receptor antagonist for the preparation of a pharmaceutical composition is provided treating myocardial ischemia (including angina pectoris, infarction, angioplasty, etc.) in mammalian species. The composition may he administered systemically, such as orally or parenterally.

Any serotonin receptor antagonist may be employed as disclosed herein. Examples of such serotonin receptor antagonists are disclosed in R.A. Glennon, Serotonin Receptors: Clinical Implications *Neuroscience and Behavioral Reviews*, 14 35-47, **1990**; and 5-Hydroxytryptamine Receptors, *Pharmacological Reviews*, vol. 44, No. 3, **1992**, which are incorporated by reference herein. Preferred serotonin receptor antagonists include cinanserin, ketanserin and LY 53857.

The compounds of formula I can be formulated for use in compositions such as tablets, capsules or elixirs for oral administration, or in sterile solutions or suspensions for parenteral administration. The compounds of formula I may also be administered via transdermal patch or nasal inhalation solutions. About 10 to 500 milligrams of a compound of formula I is compounded with physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

The dose administered must be carefully adjusted according to age, weight and condition of the patient, as well as the route of administration, dosage form and regimen and the desired result.

The compositions described above may be administered in the dosage forms as described above in single or divided doses of one to four times daily.

Many of the active substances described above form commonly known, pharmaceutically acceptable salts such as alkali metal and other common basic salts or acid addition salts, etc. References to the base substances are therefore intended to include those common salts known to be substantially equivalent to the parent compound.

Figure 1 is a graph showing the effect of the serotonin receptor antagonists ketanserin, cinanserin and LY 53857 on time to contracture (percent change relative to vehicle) in globally ischemic rat hearts.

Figures 2A to 2C are graphs showing the effect of the serotonin receptor antagonists cinanserin (Cinan), ketanserin (Ket) and LY 53,857 respectively on cumulative LDH release during reperfusion following 25 minutes of global ischemia in isolated rat hearts. All compounds significantly reduced LDH release (*, $p < 0.05$) compared to vehicle. Ketanserin given only during reperfusion (Reper) did not cause a reduction in LDH release.

Figure 3 is a graph showing the effect of serotonin (S) on time to contracture in globally ischemic, isolated rat hearts. Serotonin significantly reduced time to contracture compared to vehicle (*, $p < 0.05$) at 30 and 100$\mu$M. Ketanserin (Ket) and cinanserin (Cin) abolished the pro-ischemic effect of serotonin.

Figure 4 is a graph showing the effect of 150 (i.p.) parachlorophenylalanine (PCPA) pretreatment on time to contracture in globally ischemic rat hearts. Parachlorophenylalanine significantly increased time to contracture in these hearts when compared to vehicle (*, $p < 0.05$).

Figures 5A to 5D are graphs showing the effect of prazosin, ketanserin, cinanserin and LY 53857, respectively, on methoxamine-induced contraction in rat aortic rings.

The following examples and preparations describe the manner and process of making and using the preferred embodiments of the invention and are illustrative rather than limiting. It should be understood that there may be other embodiments which fall within the spirit and scope of the invention as defined by the claims appended hereto.

## Example

To determine the effect of serotonin receptor agonists and antagonists, male Sprague-Dawley rats (450-550 g) were prepared as described below. The rats were anesthetized using 100 mg/kg sodium pentobarbital (i.p.). They were intubated and then treated with i.v. heparin (1000 U/kg). While being mechanically ventilated, their hearts were perfused *in situ* via retrograde cannulation of the aorta. The hearts were then excised and sickly moved to a Langendorff apparatus where they were perfused with Krebs-henseleit bicarbonate buffer solution ((mM): 112 NaCl, 25 $NaHCO_3$, 5 KCl, 1.2 $MgSO_4$, 1 $KH_2PO_4$, 1.25 $CaCl_2$, 2 pyruvate and 11.5 dextrose bubbled with 95% $O_2$ - 5% $CO_2$) at a constant perfusion pressure (85 mm Hg). A water-filled latex balloon attached to a metal cannula was then inserted into the left ventricle and connected to a Statham pressure transducer for measurement of left ventricular pressure. The hearts were allowed to equilibrate for 15 min, at which time end diastolic pressure (EDP) was adjusted to 5 mm Hg and this balloon volume was maintained for the duration of the experiment. Pre-ischemia or pre-drug function, heart rate and coronary flow (extracorporeal electromagnetic flow probe) were then measured. Left ventricular developed pressure (LVDP) was calculated from the difference between left ventricular peak systolic pressure and EDP. Cardiac temperature was maintained throughout the experiment by submerging the hearts in 37°C buffer which was allowed to accumulate in a stoppered, heated chamber.

The model of global ischemia in isolated rat hearts was designed such that any protective effects observed are generally due to a direct cardioprotective effect and not to effects on cornonary or peripheral vascular tone. Also, because the hearts are perfused with an oxygenated buffer solution, platelets or the normal components of an inflammatory response are probably not important mediators of the ischemic/reperfusion injury observed in this model. It was found that agents which exert their protective effects directly on ischemic myocardium are generally efficacious in this model and such agents include calcium antagonists, ATP-sensitive potassium channel openers and calmodulin inhibitors. Agents which exert their protective effects either through their effects on the peripheral or coronary vasculature such as nitrates or agents which act on blood or act on some component of the inflammatory response such as thromboxane $A_2$ receptor antagonists are generally ineffective in this model.

Effect of 5HT receptor agonists and antagonists

The hearts were divided into the following groups: 1. Vehicle (0.1% DMSO) treatment beginning 10 minutes before the onset of ischemia (n = 8). 2. 0.3-10 $\mu$M ketanserin treatment beginning 10 minutes before the onset of ischemia (n = 4-5 per group). 3. 10 $\mu$M ketanserin given only during reperfusion (n = 4). 4. 0.1-10 $\mu$M cinanserin treatment beginning 10 minutes before global ischemia (n = 4-10). 5. 1-10 $\mu$M LY 53857 treatment beginning 10 minutes before the onset of ischemia (n = 4 per group). After 10 minutes of vehicle or drug pretreatment, the hearts were subjected to 25 minutes of total global ischemia which was initiated by shutting off the perfusate flow. Reperfusion was begun (without drug except in the case of group 3 above) and allowed to continue for 30 minutes. At this time, final measurements of EDP, LVDP and coronary flow were obtained. Cumulative LDH relase was measured in the reperfusate and was used as the index of cellular and membrane viability. In all hearts, the time to contracture during ischemia was measured and used as an index of cardioprotection during ischemia. Time to contracture is defined as the time (minutes) necessary for the first 5 mm Hg increase in EDP to occur during global ischemia. The data were expressed as the percent change in time to contracture from vehicle. The potency was expressed as the concentration ($\mu$M) of the respective drug which increased the time to contracture 25% above vehicle group values ($EC_{25}$).

In another series of hearts, the effect of 5HT on the severity of ischemia was determined. The groups were divided as follows: 1. 1-100 $\mu$M 5HT was given 10 minutes before ischemia (n = 4 per group). 2. 30 $\mu$M 5HT + 3 $\mu$M ketanserin, both given 10 minutes before ischemia (n = 4). 3. 30 $\mu$M 5HT + 30 $\mu$M cinanserin, both given 10 minutes before the onset of ischemia (n = 4). 4. Vehicle (0.1% DMSO) given 10 minutes before ischemia (n = 5). The hearts were subjected to global ischemia as described above and time to contracture determined. Reperfusion function and LDH release were not determined in this group.

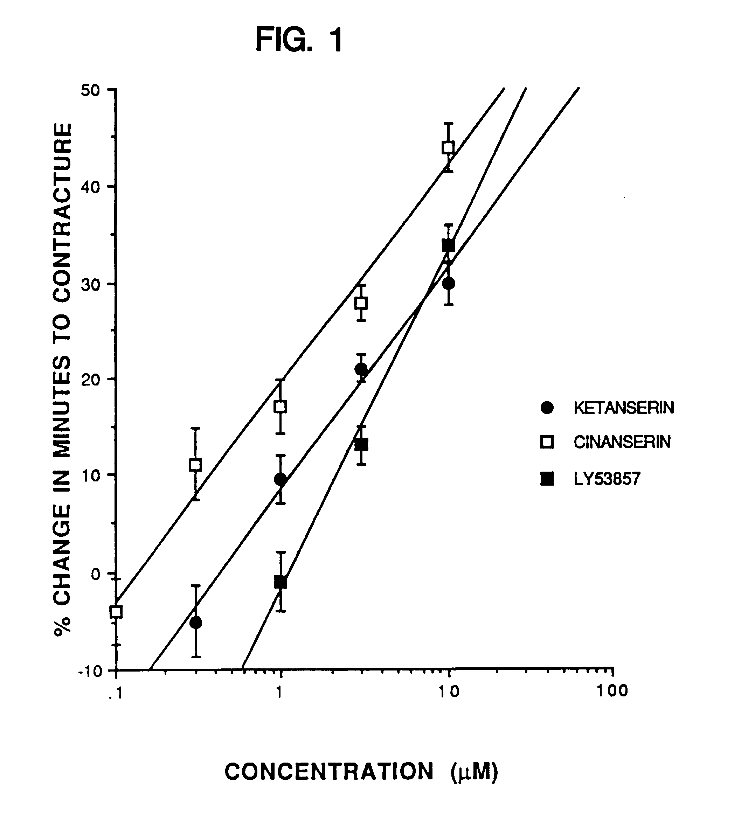

The effects of the $5HT_2$ antagonists cinanserin, ketanserin and LY 53857 on time to contracture during global ischemia are shown in Figure 1. These data are expressed as the percent change in the time necessary for the first 5 mm Hg change in EDP relative to vehicle. As can be seen in this figure, all three compounds increased time to contracture in a concentration dependent manner. Cinanserin was the most potent in increasing time to contracture with an $EC_{25}$ of 1.6 $\mu$M, followed by ketanserin ($EC_{25}$ = 5.5 $\mu$M) and LY 53857 ($EC_{25}$ = 6.1 $\mu$M).

The effect of the $5HT_2$ antagonists on pre-ischemic and post-ischemic cardiac function is shown in Tables 1 to 3. Table 1 shows the effects of cinanserin on cardiac function and coronary flow before and after Ischemia in isolated rat hearts.

## TABLE 1
## Pre-Ischemia

|  | Pre-Drug | Post-Drug | 30 Min Post-Reperfusion |
|---|---|---|---|
| **HR (beats/min)** | | | |
| Vehicle | 289±7 | 275±9 | 259±10 |
| 0.1 μM Cinanserin | 267±17 | 254±18 | 251±9 |
| 0.3 μM Cinanserin | 285±8 | 258±6 | 260±42 |
| 1 μM Cinanserin | 255±22 | 234±10 | 263±7 |
| 3 μM Cinanserin | 294±7 | 271±8 | 280±4 |
| 10 μM Cinanserin | 264±12 | 184±29[a,b] | 237±12 |
| | | | |
| **LVDP (mmHg)** | | | |
| Vehicle | 140±3 | 140±2 | 31±4[a] |
| 0.1 μM Cinanserin | 143±3 | 126±7 | 30±8[a] |
| 0.3 μM Cinanserin | 139±2 | 135±5 | 65±11[a,b] |
| 1 μM Cinanserin | 144±9 | 141±5 | 77±6[a,b] |
| 3 μM Cinanserin | 136±6 | 123±6 | 78±9[a,b] |
| 10 μM Cinanserin | 144±3 | 133±6 | 73±6[a,b] |
| | | | |
| **Coronary Flow (mL/min/g)** | | | |
| Vehicle | 17±1 | 16±1 | 12±1[a] |
| 0.1 μM Cinanserin | 18±1 | 15±1 | 15±1 |
| 0.3 μM Cinanserin | 21±2 | 19±2 | 19±1[b] |
| 1 μM Cinanserin | 22±3 | 20±3 | 20±3[b] |
| 3 μM Cinanserin | 21±3 | 20±2 | 20±2[b] |
| 10 μM Cinanserin | 17±1 | 15±1 | 12±1[a] |

All values are mean ±SE; HR=Heart Rate;

LVDP = Left Ventricular Developed Pressure

[a] Significantly different from its respective predrug value (P<0.05)

[b] Significantly different from its respective vehicle groups (P<0.05)

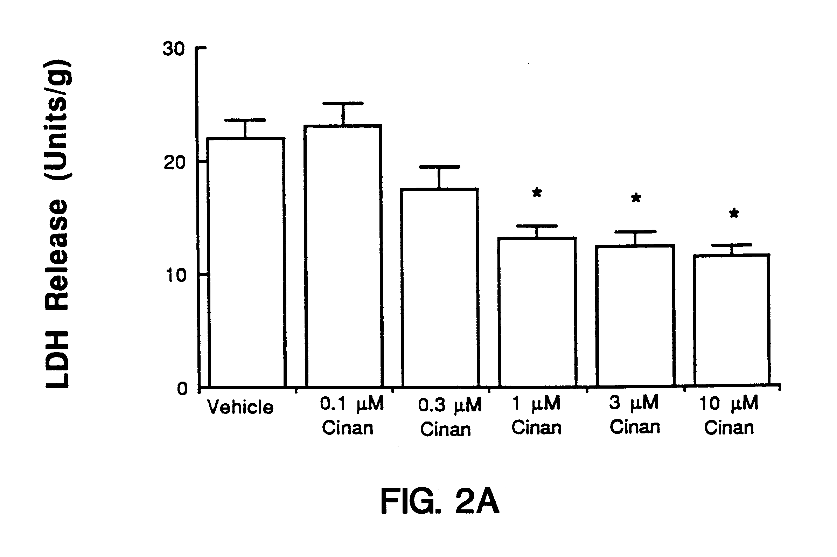

As shown in Table 1 above, cinanserin had little effect on preischemic heart rate or LVDP. Only at the 10 μM concentration was significant bradycardia observed. Despite the bradycardia seen at this concentration, no significant effect on LVDP was found. No significant effect on pre-ischemic coronary flow was seen for any concentration of cinanserin tested, although a slight reduction was observed at several concentrations. Recovery of cardiac function was also measured at 30 minutes after the initiation of reperfusion. In vehicle treated hearts, significant reperfusion contractile dysfunction was observed, indicating severe ischemic/reperfusion injury. Cinanserin significantly improved reperfusion LVDP at 0.3 μM and higher concentrations when compared to vehicle controls, although this effect did not appear to be clearly concentration related. Coronary flow was significantly reduced (relative to pre-ischemic baseline values) during reperfusion in vehicle treated animals and cinanserin significantly improved reflow at cardioprotective concentrations, except at the 10 μM concentration. In Figure 2A, the effect of cinanserin on reperfusion LDH release is shown. Significant reductions in LDH were observed for 1-10 μM cinanserin compared to vehicle. The reductions in LDH release were not found to be concentration related. Nonischemic hearts did not release LDH and historically, LDH has never been found to be released from isolated rat hearts which were not ischemic within the time frame of the experiments.

Cardiac function and coronary flow data for ketanserin and LY 53857 are shown in Tables 2 and 3.

TABLE 2

| Pre-Ischemia | | | |
|---|---|---|---|
| HR (beats/min) | Pre-Drug | Post-Drug | 30 Min Post-Reperfusion |
| Vehicle | 289±7 | 275±9 | 259±10[a] |
| 0.3 $\mu$M Ketanserin | 291±2 | 275±6 | 266±18[a] |
| 1 $\mu$M Ketanserin | 311±16 | 272±11 | 243±35[a] |
| 3 $\mu$M Ketanserin | 310±13 | 244±16[a] | 246±22[a] |
| 10 $\mu$M Ketanserin | 290±6 | 196±19[a,b] | 220±22[a] |
| 3 $\mu$M Ketanserin(Reper) | 292±15 | 285±11 | 196±14[a,b] |
| LVDP (mmHg) | | | |
| Vehicle | 140±3 | 140±2 | 31±4[a] |
| 0.3 $\mu$M Ketanserin | 146±5 | 141±10 | 20±5[a] |
| 1 $\mu$M Ketanserin | 130±6 | 128±4 | 29±5[a] |
| 3 $\mu$M Ketanserin | 128±8 | 127±9 | 53±7[a,b] |
| 10 $\mu$M Ketanserin | 135±5 | 128±5 | 66±4[a,b] |
| 3 $\mu$M Ketanserin(Reper) | 134±4 | 130±5 | 33±6[a] |
| Coronary Flow (mL/min/g) | | | |
| Vehicle | 17±1 | 16±1 | 12±1[a] |
| 0.3 $\mu$M Ketanserin | 20±2 | 16±2 | 13±2[a] |
| 1 $\mu$M Ketanserin | 19±1 | 15±1 | 10±1[a] |
| 3 $\mu$M Ketanserin | 19±1 | 15±1 | 13±1[a] |
| 10 $\mu$M Ketanserin | 19±1 | 14±2 | 13±2[a] |
| 3 $\mu$M Ketanserin(Reper) | 19±1 | 17±2 | 14±1[a] |
| For Tables 2 and 3, all values are mean ±SE; HR = Heart Rate; LVDP = Left Ventricular Developed Pressure | | | |

[a] Significantly different from its respective predrug value (P<0.05)
[b] Significantly different from its respective vehicle groups (P<0.05)

TABLE 3

| Pre-Ischemia | | | |
|---|---|---|---|
| HR (beats/min) | Pre-Drug | Post-Drug | 30 Min Post-Reperfusion |
| Vehicle | 289±7 | 275±9 | 259±10 |
| 1 $\mu$M LY 53857 | 270±5 | 257±6 | 230±25 |
| 3 $\mu$M LY 53857 | 274±5 | 265±8 | 240±13 |
| 10 $\mu$M LY 53857 | 288±10 | 252±12[a] | 250±4 |
| LVDP (mmHg) | | | |
| Vehicle | 140±3 | 140±2 | 31±4[a] |
| 1 $\mu$M LY 53857 | 141±4 | 132±6 | 23±5[a] |
| 3 $\mu$M LY 53857 | 148±6 | 144±6 | 73±7[a,b] |
| 10 $\mu$M LY 53857 | 130±5 | 123±5 | 86±6[a,b] |
| Coronary Flow (mL/min/g) | | | |
| Vehicle | 17±1 | 16±1 | 12±1[a] |
| 1 $\mu$M LY 53857 | 17±1 | 15±1 | 12±1[a] |
| 3 $\mu$M LY 53857 | 17±1 | 17±1 | 13±1[a] |
| 10 $\mu$M LY 53857 | 20±3 | 21±3 | 15±3 |
| For Tables 2 and 3, all values are mean ±SE; HR = Heart Rate; LVDP = Left Ventricular Developed Pressure | | | |

[a] Significantly different from its respective predrug value (P<0.05)
[b] Significantly different from its respective vehicle groups (P<0.05)

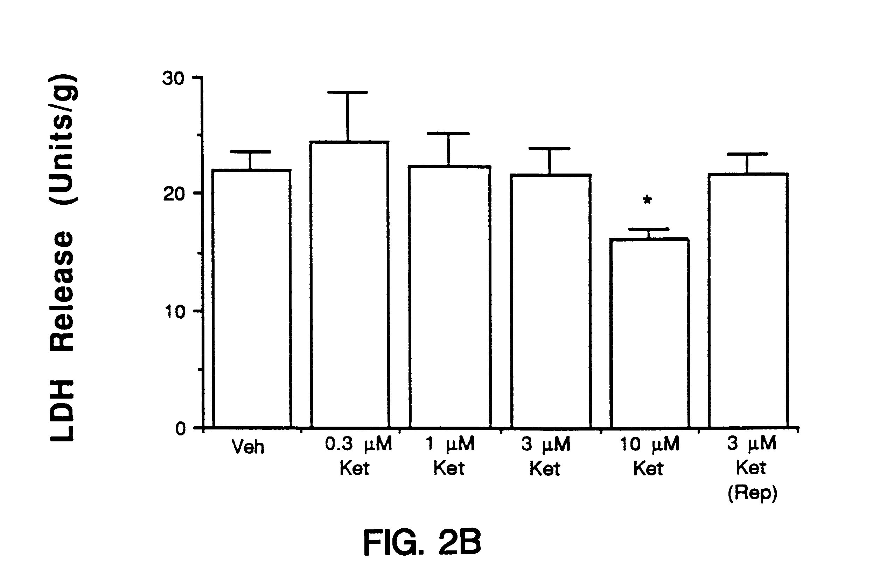

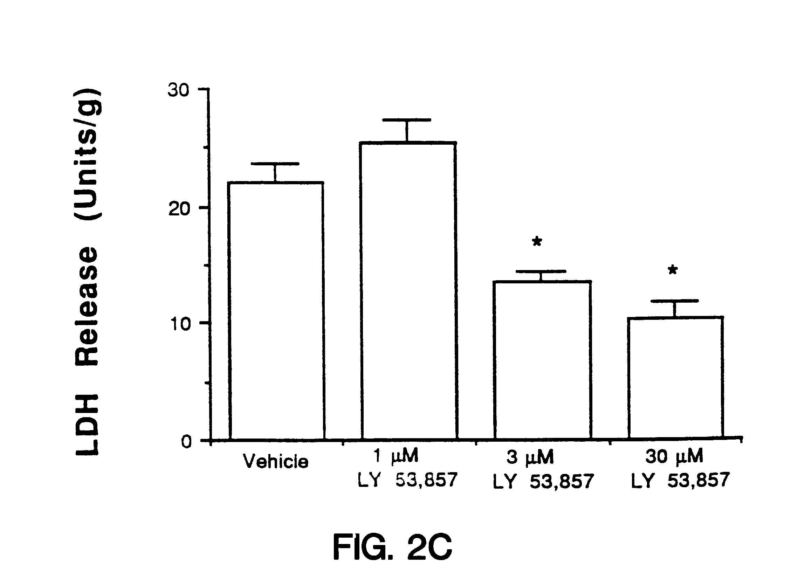

Neither ketanserin nor LY 53857 exerted marked pre-ischemic effects on cardiac function. Ketanserin caused bradycardia at the 3 and 10 $\mu$M concentrations without affecting LVDP. Ketanserin seemed to exert a moderate coronary constrictor effect in nonischemic hearts while LY 53857 was devoid of significant effects. Both compounds significantly improved reperfusion LVDP starting at 3 $\mu$M. Reperfusion flow was not improved by ketanserin nor by LY 53857. LDH data for ketanserin and LY 53857 are shown in Figures 2B and 2C, respectively. As can be seen in these figures, ketanserin only significantly reduced LDH relase at the 10 $\mu$M concentration while LY 53857 significantly reduced it at 3 and 10 $\mu$M concentrations. Ketanserin was also given only during reperfusion at 3 $\mu$M and no protective effect was observed for any variable measured.

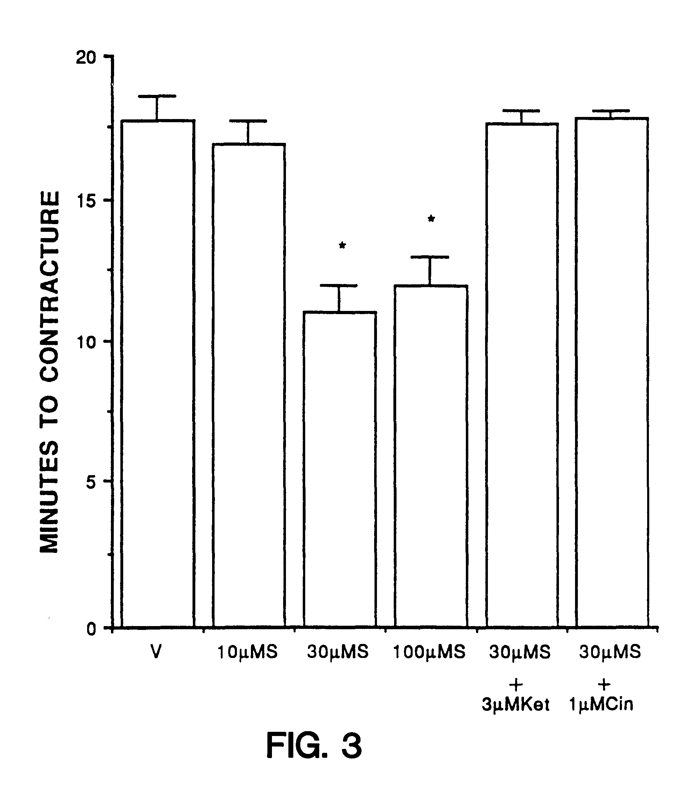

The effect of 5HT on time to contracture is shown in Figure 3. 5HT was found to significantly decrease time to contracture at >30 $\mu$M, indicating a pro-ischemic effect. The 1 and 10 $\mu$M concentrations were without any measurable effect on the severity of ischemia (data for 1 $\mu$M 5HT is not shown in Figure 3). The $5HT_2$ receptor antagonists cinanserin and ketanserin completely abolished this pro-ischemic effect. The effect of 5HT on pre-ischemic cardiac function and coronary flow is shown in Table 4.

## TABLE 4

|  | Pre-Drug | Post-Drug |
|---|---|---|
| **HR (beats/min)** | | |
| Vehicle | 302±15 | 295±13 |
| 1 μM 5HT | 273±7 | 276±5 |
| 10 μM 5HT | 300±7 | 294±11 |
| 30 μM 5HT | 281±15 | 319±20 |
| 100 μM 5HT | 298±10 | 369±19[a,b] |
| 30 μM 5HT + 3 μM Ketanserin | 270±16 | 335±17[a] |
| 30 μM 5HT + 1 μM Cinanserin | 297±9 | 340±11[a,b] |
| | | |
| **LVDP (mmHg)** | | |
| Vehicle | 122±5 | 121±7 |
| 1 μM 5HT | 145±4 | 141±3 |
| 10 μM 5HT | 135±4 | 132±7 |
| 30 μM 5HT | 117±5 | 103±11 |
| 100 μM 5HT | 121±8 | 88±8[a,b] |
| 30 μM 5HT + 3 μM Ketanserin | 141±4 | 130±2 |
| 30 μM 5HT + 1 μM Cinanserin | 139±7 | 122±5 |
| | | |
| **Coronary Flow (mL/min/g)** | | |
| Vehicle | 19±2 | 18±2 |
| 1 μM 5HT | 16±1 | 16±1 |
| 10 μM 5HT | 19±2 | 20±2 |
| 30 μM 5HT | 16±1 | 14±1 |
| 100 μM 5HT | 16±2 | 15±3 |
| 30 μM 5HT + 3 μM Ketanserin | 18±2 | 18±1 |
| 30 μM 5HT + 1 μM Cinanserin | 19±1 | 18±1 |

All values are mean ±SE; HR=Heart Rate;

LVDP = Left Ventricular Developed Pressure

[a] Significantly different from its respective predrug value ($P<0.05$)

[b] Significantly different from its respective vehicle groups ($P<0.05$)

5HT increased heart rate in a concentration dependent manner. Along with tachycardia, 5HT reduced LVDP, although this was only observed at the 100 μM concentration. Typically, slight negative inotropy in rat hearts subjected to tachycardia is observed. Ketanserin and cinanserin only partially abolished the tachycardia. No significant effect on coronary flow was observed.

Effect of Parachlorophenylalanine on Ischemic Hearts

To determine if endogenous 5HT is mediating a pro-ischemic effect in isolated hearts, rats, prepared as described below were treated with parachlorophenylalanine. Parachlorophenylalanine depletes brain and peripheral 5HT by inhibition of tryptophan hydroxylase, the rate limiting step in 5HT synthesis. The treatment regimen of parachlorophenylalanine use was found previously to significantly deplete 5HT. Male Sprague-Dawely rats (450-550 g) were injected with i.p. 105 mg/kg parachlorophenylalanine (n = 10) or its vehicle (water, n = 6) on three consecutive days. On the fourth day, the rats were anesthetized with 100 mg/kg sodium pentobarbital. The hearts were then excised and moved to an isolated heart apparatus as described above. The hearts were then subjected to 25 minutes to global ischemia and 30 minutes of reperfusion. Time to contracture, recovery of contractile function, coronary flow and cumulative LDH release were measured as described above.

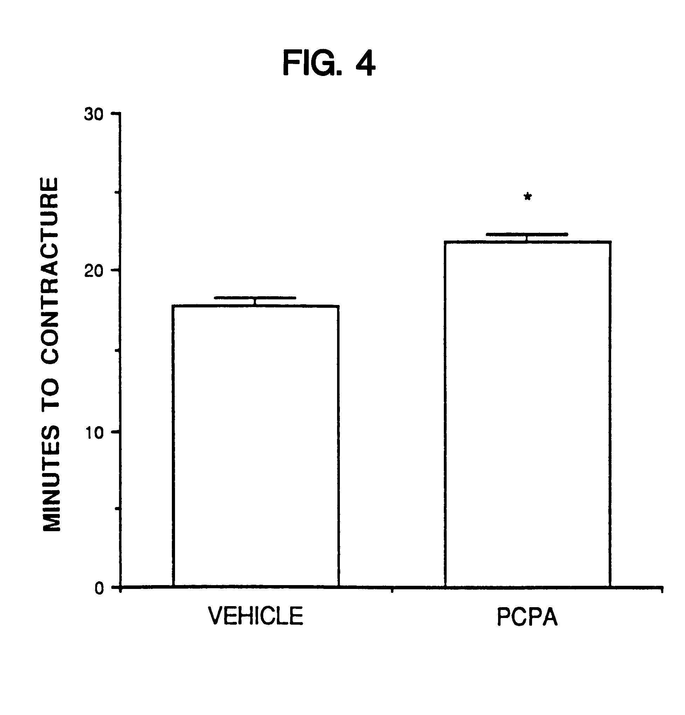

Time to contracture data are shown in Figure 4. As can be seen parachlorophenylalanine (PCPA) significantly incareased the time to contracture in these hearts, indicating a protective effect. The effect of parachlorophenylalanine on pre-ischemic and post-ischemic cardiac function and coronary flow is shown in Table 5.

## TABLE 5

|  | Pre-Ischemia | 30 Min. Post-Ischemia |
|---|---|---|
| **HR (beats/min)** | | |
| Vehicle | $257\pm10$ | $200\pm35^{a}$ |
| PCPA | $271\pm8$ | $275\pm8^{b}$ |
| **LVDP (mmHg)** | | |
| Vehicle | $139\pm3$ | $18\pm1^{a}$ |
| PCPA | $128\pm11$ | $60\pm7^{a,b}$ |
| **Coronary Flow (mL/min/g)** | | |
| Vehicle | $16\pm1$ | $9\pm1^{a}$ |
| PCPA | $18\pm1$ | $18\pm2^{b}$ |
| **LDH Release (u/g)** | | |
| Vehicle | – | $21\pm2$ |
| PCPA | – | $15\pm2^{b}$ |

All values are mean $\pm$SE; HR=Heart Rate;

LVDP = Left Ventricular Developed Pressure

[a] Significantly different from its respective predrug value ($P<0.05$)

[b] Significantly different from its respective vehicle groups ($P<0.05$)

The reperfusion bradycardia and contractile dysfunction observed in vehicle treated hearts was significantly attenuated by parachlorophenylalanine. Also shown in this Table 5 is LDH release during reperfusion, which was slightly, but significantly reduced by parachlorophenylalanine.

Effect of 5HT$_2$ Antagonists on alpha$_1$ Adrenoceptors in Vascular Smooth Muscle

In order to determine the alpha$_1$ blocking potency of cinanserin, ketanserin and LY 53857, their effect on methoxamine induced aortic constriction was determined. Male Sprague-Dawley rats were sacrificed using $CO_2$. Aortic rings (3 mm in width) were cut, denuded of endothelium by gently rolling the rings on moistened filter paper, and mounted on stainless steel hooks in 20 mL muscle chambers containing oxygenated physiological salt solution (PSS) of the following composition (in mM): 118.4 NaCl, 4.7 KCl, 1.2 $MgSO_4$, 1.2 $KH_2PO_4$, 1.9 $CaCl_2$, 25.0 $NaHCO_3$, 10.1 D-glucose, and 0.01 mM $Na_2$EDTA maintained at 37°C. The hooks were connected to Grass FT03C transducers for recording of isometric force development. The rings were stretched to 2 g preload during the equilibration period. During equilibration the rings were periodically stimulated with 24 mM KCl to determine contractility. The effectiveness of endothelial removal was confirmed by the absence of relaxation by 1 $\mu$M acetylcholine of a contraction in response to 0.01 $\mu$M phenylephrine.

Various concentrations of the test compounds were then added to individual baths and allowed a 45 minute incubation before cumulative concentration response curves for methoxamine (1 nM-30 $\mu$M) were obtained. Solvent controls were run in parallel. The compounds tested were 0.3-10 nM prazosin (n = 4-8 per group), 0.1-10 $\mu$M ketanserin (n = 4-8 per group), 1-100 $\mu$M cinanserin (n = 4 per group), or 10-100 $\mu$M LY 53857 (n = 4 per group). The data were plotted as the mean±SE of at least 4 tissues from different animals.

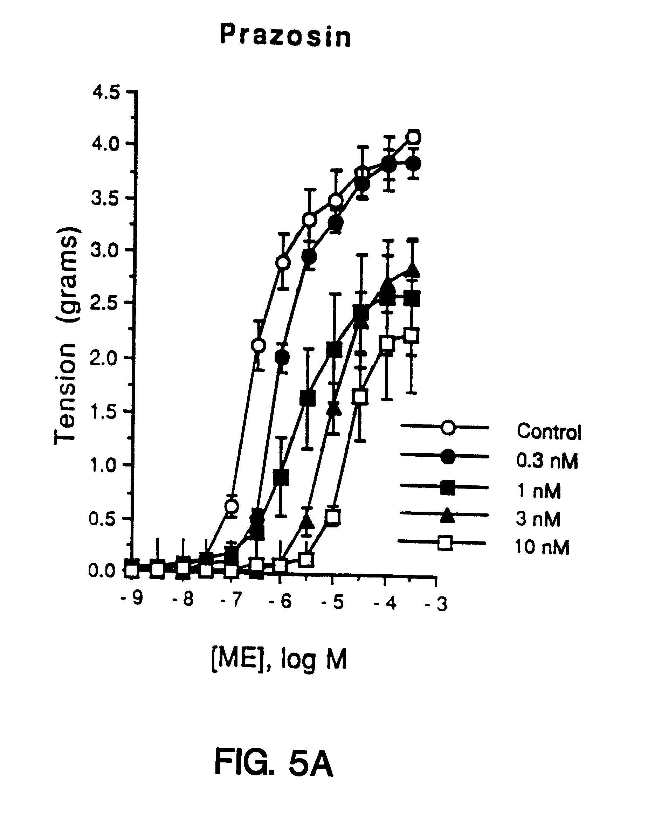

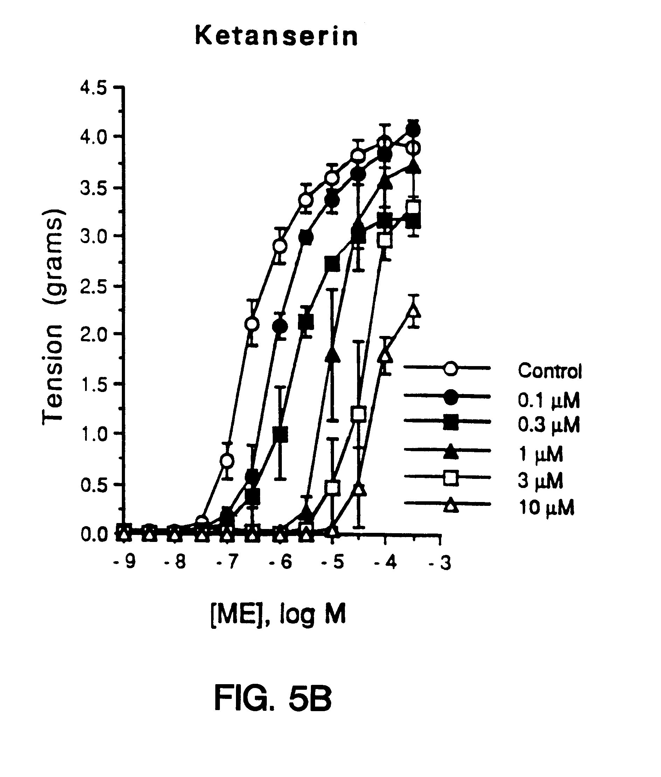

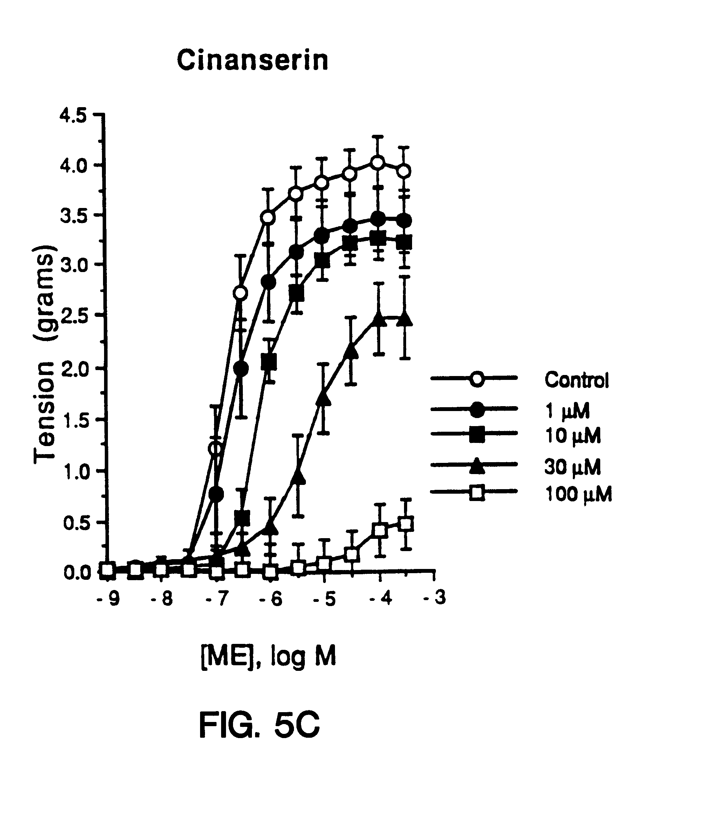

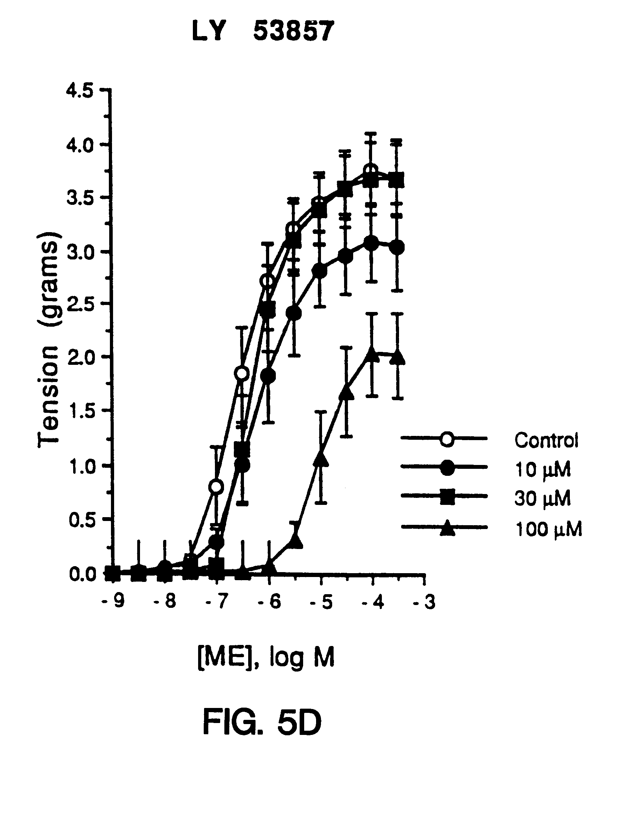

As shown in Figure 5A, prazosin shifted the methoxamine-tension curve to the right starting at a concentration of 1 nM. The rightward shift did not appear to be clearly competitive. Ketanserin (Figure 5B) significantly shifted the curve as well, although this was significant starting at the 0.3 $\mu$M concentration. Ketanserin appeared to inhibit methoxamine-induced contractions in a competitive fashion. Both cinanserin and LY 53857 (Figures 5C and 5D, respectively) produced rightward shifts, but this was not clearly concentration related. Neither compound seemed to have an effect on methoxamine-induced contractions until 30-100 $\mu$M concentrations were reached and then significant inhibition was observed, although maximal effects were reduced.

Despite a lack of blood perfusing the hearts, cinanserin, ketanserin and LY 53857 all significantly protected the hearts in this study. Cinanserin appeared to be slightly more potent as a cardioprotective agent relative to the other compounds in terms of their ability to increase time to contracture during ischemia, although in general their $EC_{25}$ values were similar. All compounds increased time to contracture indicating that at least some of their protective effects occured during ischemia per se. All of the $5HT_2$ antagonists improved the recovery of contractile function during reperfusion, but infusion of ketanserin only during reperfusion did not result in a protective effect, further suggesting that the protective effects of $5HT_2$ antagonists occur during the global ischemia itself. LY 53857, an octahydroquinoline derivative is structurally distinct from the piperdine $5HT_2$ antagonists cinanserin and ketanserin and is still equivalent in antiischemic potency.

It was also determined if depletion of 5HT using parachlorophenylalanine could result in cardioprotection. Parachlorophenylalanine exerted significant cardioprotective effects which were similar in magnitude to that observed for the $5HT_2$ receptor antagonists. This is further evidence that antagonism of 5HT activity is the mechanism of action of the agents tested herein. These data agree with previous findings in which rat hearts have been found to have measurable levels of 5HT and that they can accumulate 5HT at neuronal and nonneuronal sites.

In summary, $5HT_2$ receptor antagonism in isolated rat hearts results in significant cardioprotection which seems to occur during ischemia and does not attenuate reperfusion damage.

**Claims**

1. Use of a serotonin receptor antagonist for the preparation of a pharmaceutical composition useful in treating myocardial ischemia.

2. The use as recited in Claim 1 wherein said serotonin receptor antagonist is a $5HT_2$ receptor antagonist.

3. The use as recited in Claim 1 wherein said serotonin receptor antagonist is cinanserin.

4. The use as recited in Claim 1 wherein said serotonin receptor antagonist is ketanserin.

5. The use as recited in Claim 1 wherein said serotonin receptor antagonist is LY 53857.

FIG. 1

LDH Release (Units/g)

FIG. 2A

LDH Release (Units/g)

FIG. 2B

FIG. 2C

LDH Release (Units/g)

Vehicle

1 μM LY 53,857

3 μM LY 53,857

30 μM LY 53,857

FIG. 3

# FIG. 4

# Prazosin

FIG. 5A

FIG. 5B

**Cinanserin**

FIG. 5C

LY 53857

FIG. 5D